# EUROPEAN PATENT APPLICATION

(11) **EP 1 541 695 A1**
(43) Date of publication of application: **15.06.2005**
(21) Application number: 03028074.7
(22) Date of filing: 09.12.2003
(51) Int. Cl.: C12Q 1/68

(54) **Use of a mutation in the BRAF gene for the determination of the malignancy of melanoma cells**

(71) Applicant: Nanogen Recognomics GmbH, 65926 Frankfurt am Main (DE)
(72) Inventor: Kappel, Andreas, Dr., 61462 Königstein (DE)
(74) Representative: Ackermann, Joachim, Dr.

(57) **Abstract**

The invention relates to the use of oligonucleotide probes comprising exon 15 of the BRAF gene or a part thereof comprising codon 599 for the detection of the malignancy of melanoma cells.

SEQUENCE LISTING

<110> Nanogen Recognomics GmbH

<120> Use of a mutation in the BRAF gene for the
      determination of the malignancy of melanoma cells

<130> 202nr05.ep

<140>
<141>

<160> 7

<170> PatentIn Ver. 2.1

<210> 1
<211> 119
<212> DNA
<213> Homo sapiens

<400> 1
atatatttct tcatgaagac ctcacagtaa aaataggtga ttttggtcta gctacagtga 60
aatctcgatg gagtgggtcc catcagtttg aacagttgtc tggatccatt ttgtggatg  119


<210> 2
<211> 2510
<212> DNA
<213> Homo sapiens

<400> 2
cgcctcccgg ccccctcccc gcccgacagc ggccgctcgg gccccggctc tcggttataa 60
gatggcggcg ctgagcggtg gcggtggtgg cggcgcggag ccgggccagg ctctgttcaa 120
cggggacatg gagcccgagg ccggcgccgg ccggcccgcg gcctcttcgg ctgcggaccc 180
tgccattccg gaggaggtgt ggaatatcaa acaaatgatt aagttgacac aggaacatat 240
agaggcccta ttggacaaat ttggtgggga gcataatcca ccatcaatat atctggaggc 300
ctatgaagaa tacaccagca agctagatgc actccaacaa agagaacaac agttattgga 360
atctctgggg aacggaactg atttttctgt ttctagctct gcatcaatgg ataccgttac 420
atcttcttcc tcttctagcc tttcagtgct accttcatct ctttcagttt ttcaaaatcc 480
cacagatgtg gcacggagca accccaagtc accacaaaaa cctatcgtta gagtcttcct 540
gcccaacaaa cagaggacag tggtacctgc aaggtgtgga gttacagtcc gagacagtct 600
aaagaaagca ctgatgatga gaggtctaat cccagagtgc tgtgctgttt acagaattca 660
ggatggagag aagaaaccaa ttggttggga cactgatatt tcctggctta ctggagaaga 720
attgcatgtg gaagtgttgg agaatgttcc acttacaaca cacaactttg tacgaaaaac 780
gtttttcacc ttagcatttt gtgacttttg tcgaaagctg cttttccagg gtttccgctg 840
tcaaacatgt ggttataaat ttcaccagcg ttgtagtaca gaagttccac tgatgtgtgt 900
taattatgac caacttgatt tgctgtttgt ctccaagttc tttgaacacc acccaatacc 960

acaggaagag gcgtccttag cagagactgc cctaacatct ggatcatccc cttccgcacc 1020
cgcctcggac tctattgggc cccaaattct caccagtccg tctccttcaa aatccattcc 1080
aattccacag cccttccgac cagcagatga agatcatcga aatcaatttg ggcaacgaga 1140
ccgatcctca tcagctccca atgtgcatat aaacacaata gaacctgtca atattgatga 1200
cttgattaga gaccaaggat ttcgtggtga tggaggatca accacaggtt tgtctgctac 1260
cccccctgcc tcattacctg gctcactaac taacgtgaaa gccttacaga aatctccagg 1320
acctcagcga gaaaggaagt catcttcatc ctcagaagac aggaatcgaa tgaaaacact 1380
tggtagacgg gactcgagtg atgattggga gattcctgat gggcagatta cagtgggaca 1440
aagaattgga tctggatcat ttggaacagt ctacaaggga aagtggcatg gtgatgtggc 1500
agtgaaaatg ttgaatgtga cagcacctac acctcagcag ttacaagcct tcaaaaatga 1560
agtaggagta ctcaggaaaa cacgacatgt gaatatccta ctcttcatgg gctattccac 1620
aaagccacaa ctggctattg ttacccagtg gtgtgagggc tccagcttgt atcaccatct 1680
ccatatcatt gagaccaaat ttgagatgat caaacttata gatattgcac gacagactgc 1740
acagggcatg gattacttac acgccaagtc aatcatccac agagacctca agagtaataa 1800
tatatttctt catgaagacc tcacagtaaa aataggtgat tttggtctag ctacagtgaa 1860
atctcgatgg agtgggtccc atcagtttga acagttgtct ggatccattt tgtggatggc 1920
accagaagtc atcagaatgc aagataaaaa tccatacagc tttcagtcag atgtatatgc 1980
atttgggatt gttctgtatg aattgatgac tggacagtta ccttattcaa acatcaacaa 2040
cagggaccag ataattttta tggtgggacg aggatacctg tctccagatc tcagtaaggt 2100
acggagtaac tgtccaaaag ccatgaagag attaatggca gagtgcctca aaaagaaaag 2160
agatgagaga ccactctttc cccaaattct cgcctctatt gagctgctgg cccgctcatt 2220
gccaaaaatt caccgcagtg catcagaacc ctccttgaat cgggctggtt tccaaacaga 2280
ggattttagt ctatatgctt gtgcttctcc aaaaacaccc atccaggcag ggggatatgg 2340
tgcgtttcct gtccactgaa acaaatgagt gagagagttc aggagagtag caacaaaagg 2400
aaaataaatg aacatatgtt tgcttatatg ttaaattgaa taaaatactc tctttttttt 2460
taaggtggaa aaaaaaaaaa aaaaaaaaaa aaaaaaaaaa aaaaaaaccc 2510

<210> 3
<211> 20
<212> DNA
<213> Artificial Sequence

<220>
<223> Description of Artificial Sequence: primer

<400> 3
tagcctcaat tcttaccatc 20

<210> 4
<211> 21
<212> DNA
<213> Artificial Sequence

<220>
<223> Description of Artificial Sequence: primer

<400> 4
cataatgctt gctctgatag g                                                21

<210> 5
<211> 13
<212> DNA
<213> Artificial Sequence

<220>
<223> Description of Artificial Sequence: reporter wt

<400> 5
catcgagatt tca                                                         13

<210> 6
<211> 13
<212> DNA
<213> Artificial Sequence

<220>
<223> Description of Artificial Sequence: reporter mut

<400> 6
catcgagatt tct                                                         13

<210> 7
<211> 31
<212> DNA
<213> Artificial Sequence

<220>
<223> Description of Artificial Sequence: stabilizer

<400> 7
ctgtagctag accaaaatca cctattttta c                                     31

## Description

The present invention relates to the use of oligonucleotide probes comprising a part of the BRAF gene for the determination of the malignancy of melanoma cells and a method for the determination of the malignancy of melanoma cells.

A fast determination of the malignancy of a tumor, that in particular corresponds with the occurrence of metastasising cells, is important to provide an optimized treatment of a tumor. To establish new methods for the diagnosis of the stage of an malignant melanoma tumor the identification of specific markers correlated with the malignancy of tumor cells is desired.

The BRAF gene coding for the BRAF kinase has been suggested to be implicated in tumorigenesis almost a decade ago (S.M. Storm & U. Rapp, Toxicol. Lett. 67, 201-210, 1993). However, no clinical data have been published that demonstrate a role for BRAF in human cancers.

In a recent publication (H. Davies et al., Nature 417, 949-954, 2002), the presence of a mutation in exon 15 of the BRAF gene, wherein the valine codon is replaced by a glutamic acid codon by a mutation in codon 599 (V599E) was described. The mutation leads to a constitutive active variant of the BRAF kinase. However, only a limited number of primary malignant melanoma was examined and the melanoma were not classified according to their clinical stage.

The availability of a large panel of markers may allow a better discrimination between different clinical stages of melanocytic tumors and a more refined treatment of the tumors in the future.

It is an objection of the present invention to provide a new marker correlated with the malignancy of a tumor and its use and to provide methods for the determination of the clinical stage of melanoma using said marker.

It was found that only some biopsy samples of human malignant melanoma harbour the V599E mutation in exon 15 of the BRAF gene at a high fraction. Surprisingly, the samples of patients bearing the V599E mutation derived from malignant, in particular metastasising tumors. Samples of patients encompassing only the wildtype (a valine codon at position 599) derived from less malignant in particular not metastasising primary tumors.

Based on the correlation of the V599E mutation with the malignancy of melanoma an embodiment of the present invention is the use of an oligonucleotide probe comprising exon 15 of the BRAF gene or a part thereof comprising codon 599 or the counterstrands thereto for the detection of the malignancy of melanoma tumors or cells.

A particular exon 15 sequence of the BRAF gene is shown in **Seq ID No. 1:**

5′- AT ATA TTT CTT CAT GAA GAC CTC ACA GTA AAA ATA GGT GAT TTT
GGT CTA GCT ACA GTG AAA TCT CGA TGG AGT GGG TCC CAT CAG TTT
GAA CAG TTG TCT GGA TCC ATT TTG TGG ATG -3′ (Seq ID No. 1)

The underlined nucleotides indicates the valine codon 599 of the wildtype BRAF gene **(Seq. ID No. 2).**

Oligonucleotides comprising a sequence according to **Seq ID No.** 1 or oligonucleotides comprising a sequence complementary to **Seq ID No. 1** or a part of said sequences comprising codon 599 or an allelic variant thereof are preferred for the detection of the malignancy of melanoma cells.

In particular suitable parts of **Seq ID No. 1** encompassing codon 599 of the BRAF gene possesses preferably at least 10, better at least 20 nucleotides.

The invention is not limited to the use of oligonucleotides derived from **Seq ID No. 1**. In particular all allelic variants thereof, in particular oligonucleotides bearing a mutation in codon 599 of the BRAF gene are also encompassed. Allelic variants are defined oligonucleotides encompassed from melanoma probes, e.g. from patients, which hybridises with an oligonucleotide with **Seq ID No. 1** or its counterstand under stringent conditions. Allelic variants have usually a sequence homology of more than 60%, in particular of more than 80% to **Seq ID No. 1**. Particularly preferred are oligonucleotides with a sequence wherein the homology is at least 90%.

In an highly preferred embodiment of the invention oligonucleotides are used wherein the codon 599 (GTG, GTA, GTC or GTT) is replaced through a codon GAG or GAA coding for glutamic acid or through a codon GGG, GGA, GGC or GGT coding for aspartic acid, but also oligonucleotides with other mutations in codon 599 can be used for the present invention.

The described oligonucleotide probes can be used for an allelic association with the malignancy of melanoma tumors. This opens the possibility to create methods for genotyping the melanoma of individuals. Therefore an other embodiment of the present invention is a method for the detection of the malignancy of melanoma tumors wherein the presence of a mutation in codon 599 in exon 15 of the BRAF gene or a part thereof comprising codon 599 is determined in melanoma probes comprising oligonucleotides encompassing parts of the BRAF gene bearing codon 599 or a complementary strand thereto.

A method for the preparation of a genomic sample probe comprising nucleic acid sequences from exon 15 of the BRAF gene is described in (H. Davies et al., Nature 417, 949-954, 2002). The obtained oligonucleotide probes can be amplified by PCR using the primers **Seq. ID No. 3** and **Seq. ID No. 4.** But not only nucleic acid sequences derived from the genomic BRAF gene can be used in the present invention, also the corresponding mRNAs and cDNAs or parts thereof can be used for the determination of the malignancy of melanoma cells. Such genomic DNAs, mRNAs, cDNAs, its amplification products or parts thereof are explicitly encompassed of the term "oligonucleotide" respectively of the term "nucleic acid sequences".

The detection of a mutation in codon 599 of exon 15 of the BRAF gene can be carried out with all known methods, e.g. by sequencing the isolated or amplified oligonucleotides, by northern respectively southern blotting or by hybridising the isolated or amplified nucleic acid sequences on a biochip with suitable reporter oligonucleotides with a complementary sequence to exon 15 of the BRAF gene or a part thereof.

Suitable reporter are e.g. oligonucleotides comprising a sequence **Seq. ID No. 5** (wildtype reporter) and **Seq. ID No. 6** (mutant reporter) or a substantially homologous sequence, in particular with an homology of at least 80%, particularly preferred of at least 90%. Preferred reporter oligonucleotides are labelled. Possible well known labels are dyes, eg. Cy™ 3 or Cy™ 5 (Amersham Pharmacia), fluorophores or a radioactive labelling of oligonucleotides. The use of suitable reporter oligonucleotides for the determination of the malignancy of melanoma cells or tumors and said reporter itself are an other embodiment of the present invention.

A preferred method for the detection of malignancy of melanoma cells or tumors is characterised by the parallel hybridisation of an labelled wildtype reporter and an mutant reporter marked with a different label under stringent conditions. The genotype of the examined sample (homozygote wildtype, heterozygote mutant/wildtype, homozygote mutant/mutant) can be determined through the intensity ratio of the signals derived from the hybridised reporters.

The term "hybridise under stringent conditions" means that two oligonucleotides are capable to hybridise with one another under standard hybridisation conditions as described in Sambrook, et al. Molecular Cloning: A laboratory manual (1989), Cold Spring Harbor Laboratory Press, New York, USA. For this purpose, it is possible to use common stringent hybridization conditions (e.g. 60°C, 0.1x SSC, 0.1% SDS), e.g..

The following examples show that codon 599 of exon 15 of the BRAF gene, in particular a mutation leading to the replacement of the valine codon into a glutamic acid codon, is a valuable prognostic marker in the diagnosis of the clinical stage of tumor cells of a patient.

### Examples:

In the present examples the BRAF mutation status of malignant melanoma that were either classified as "primary tumors" or "metastasising tumors". Tumor samples were classified by the University of Wuerzburg, and DNA samples were kindly provided by this institution.

The BRAF exon 15 sequence from each sample was amplified in a PCR reaction containing 77 µl aqua (bidest), 10 µl 10x reaction buffer (Promega), 8 µl 25 mM MgCl2, 2 µl 10mM dNTPs, 1 µl Taq Polymerase (Promega), 0.6 µl 100 µM primer 5'-tagcctcaattcttaccatc-3' **(Seq. ID No. 3),** 0.6µl 100µM primer 5' biotin-cataatgcttgctctgatagg-3' **(Seq. ID No. 4),** and 1 µl genomic DNA. Amplification parameters were: 2 min at 95°C, 35 cycles (30 sec at 95°C, 20 sec at 55°C, 25 sec at 72°C) and 5 min at 72°C. The PCR amplicons were subsequently purified and addressed to a NanoChip^{TM} DNA microarray as described recently (H.A. Behrensdorf et al., Nucl. Acids Res., e64, 2002). Subsequently, the cartridge was incubated with 0.1 M NaOH for 5min, rinsed with 5ml 50mM histidine, and rinsed with 500µl high salt buffer (50 mM NaCl, 500mM NaP04 pH 7.5).

Labelled oligonucleotides that are complementary to either the wildtype BRAF sequence (wt, 5' Cy3- CAT CGA GAT TTC A -3' (Cy™ 3 labelled **Seq. ID No. 5))** or the BRAF V599E variant (mut, 5' Cy5- CAT CGA GAT TTC T -3' (Cy™ 5 labelled **Seq ID No. 6)),** and the stabilizer oligonucleotide 5'- CTG TAG CTA GAC CAA AAT CAC CTA TTT TTA C -3' (**Seq. ID No. 7)** were subsequently mixed and diluted in high salt buffer to a final concentration of 1 µM of each reporter oligonucleotide and 2µM of the stabilizer oligonucleotide . The mixture was subsequently incubated with the microarray surface for 5min.

After washing the microarray with 1 ml high salt buffer, the array was scanned for Cy™ 3 and Cy™ 5 fluorescence, respectively. Subsequently, the array was washed with 175 µl low salt buffer (50 mM NaPO₄) three times at 34°C, and scanned for Cy™ 3 and Cy™ 5 fluorescence, respectively. This washing and scanning step was repeated at 35°C, 36°C and 37°C. A robust discrimination was usually observed at 35°C or 36°C, respectively. This point was reached when the green/red ratio in a homozygous wildtype control sample was >5:1, and the green/red ratio in a homozygous mutant control sample was <1:5, with a green/red ratio in a heterozygote sample arbitrarily set to 1:1. Genotyping of the samples was then performed using the NanoChip^{TM} Workstation's software. The results are shown in the following table 1:

The analysis revealed the presence of two groups within the patients that were examined, one having a wildtype genotype, the other with a predominant mutant genotype. Surprisingly a correlation between the genotype and the malignancy of the tumor was found. The "wildtype" group (wt/wt) suffered from non-metastasising melanoma (examples 1 to 12), whereas the "mutant" group (V599E/wt; V599E/V599E) suffered from metastasising tumors (examples 13 to 29).

**Table 1:**

| **Sample No.** | **Cy™ 3 fluorescence (Red)** | **Cy™ 5 fluorescence (Green)** | **Ratio (Red: Green)** | **Robe Designation** |
|---|---|---|---|---|
| **1** | 18.7 | 862.44 | 1 : 46.12 | wt / wt |
| **2** | 1.3 | 245.75 | 1 : 189.04 | wt / wt |
| **3** | 2.65 | 417.99 | 1 : 157.73 | wt / wt |
| **4** | 18.5 | 1036.85 | 1 : 56.05 | wt / wt |
| **5** | 2.05 | 358.77 | 1: 175.01 | wt / wt |
| **6** | 0 | 208.80 | Inf. | wt / wt |
| **7** | 0 | 176.75 | Inf. | wt / wt |
| **8** | 0.85 | 202.28 | 1 : 237.98 | wt / wt |
| **9** | 0 | 170.77 | Inf. | wt / wt |
| **10** | 0.28 | 231.08 | 1 : 840.30 | wt / wt |
| **11** | 0 | 124.04 | Inf. | wt/wt |
| **12** | 1.3 | 140.89 | 1 : 108.37 | wt / wt |
| **13** | 1036.85 | 1036.85 | 1 : 1 | V599E / wt |
| **14** | 92.7 | 175.12 | 1 : 1.89 | V599E / wt |
| **15** | 16.45 | 39.28 | 1 : 2.39 | - / - |
| **16** | 1036.85 | 1036.85 | 1 : 1 | V599E / wt |
| **17** | 6.45 | 377.78 | 1 : 58.57 | wt / wt |
| **18** | 17.65 | 23.53 | 1 : 1.33 | V599E / wt |
| **19** | 6.15 | 286.50 | 1 : 46.59 | wt / wt |
| **20** | 3.95 | 144.69 | 1 : 36.63 | wt / wt |
| **21** | 237.35 | 231.08 | 1.03: 1 | V599E / wt |
| **22** | 109.35 | 124.04 | 1 : 1.13 | V599E / wt |
| **23** | 8.65 | 717.37 | 1 : 82.93 | wt / wt |
| **24** | 27.25 | 0 | Inf. | V599E / V599E |
| **25** | 0 | 158.82 | Inf. | wt / wt |
| **26** | 20.65 | 0 | Inf. | V599E / V599E |
| **27** | 16.85 | 1036.85 | 1 : 61.53 | wt / wt |
| **28** | 151.85 | 295.74 | 1 : 1.95 | V599E / wt |
| **29** | 1036.85 | 107.74 | 9.62 : 1 | V599E / V599E |

## Claims

1. The use of an oligonucleotide probe comprising exon 15 of the BRAF gene or a part thereof comprising codon 599 or the counterstrands thereto for the detection of the malignancy of melanoma cells.

2. The use of an oligonucleotide probe according to claim 1 wherein an oligonucleotide comprising the sequence **Seq. ID No. 1** or an oligonucleotide comprising a sequence complementary to **Seq ID No. 1** or a part of said sequences comprising codon 599 or an allelic variant thereof is used for the detection of the malignancy of melanoma cells.

3. The use of an oligonucleotide probe according to claim 1 or 2 wherein codon 599 is bearing a mutation.

4. The use of an oligonucleotide probe according to claim 1 or 2 wherein an codon 599 codes for an amino acid selected from the group consisting of valine (Val, V), glutamic acid (Glu, E) and aspartic acid (Asp, D)

5. The use of an oligonucleotide probe according to any one of claims 1 to 4 wherein said oligonucleotide comprises a sequence according to **Seq. ID No. 5** or **Seq. ID No. 6** or a sequence complementary to **Seq. ID No. 5 or Seq. ID No. 6** or a sequence with an homology of over 80% to said sequences.

6. A method for the detection of the malignancy of melanoma cells wherein the presence of a mutation in codon 599 in exon 15 of the BRAF gene or a part thereof comprinsing codon 599 is determined.

7. A method according to claim 6 wherein the presence of a mutation in codon 599 leading to a replacement of valine (wildtyp) into glutamic acid (Glu, E) or aspartic acid (Asp, D) is determined.

8. A method according to claim 6 or 7 wherein the detection of a mutation in codon 599 is carried out by sequencing of exon 15 of the BRAF gene or a part thereof comprising codon 599 or by hybridising exon 15 oligonucleotide of the BRAF gene or a part thereof comprising codon 599 with a reporter oligonucleotide with a sequence complementary to the exon 15 sequence of the BRAF gene or a part thereof.

9. The use of reporter oligonucleotides comprising a sequence **Seq. ID No. 5** or **Seq. ID No. 6** or a sequence with an homology of over 80% to **Seq. ID No. 5** or **Seq. ID No. 6** or a sequence complementary to said sequences for the determination of the malignancy of melanoma cells.

10. Labelled reporter oligonucleotides comprising a sequence **Seq. ID No. 5** or **Seq. ID No. 6** or a sequence with an homology of over 80% to **Seq. ID No. 5** or **Seq. ID No. 6** or a sequence complementary to said sequences.
